(19) Europäisches Patentamt
European Patent Office
Office européen des brevets

(11) **EP 1 836 959 A1**

(12) **EUROPEAN PATENT APPLICATION**

(43) Date of publication:
**26.09.2007 Bulletin 2007/39**

(51) Int Cl.:
**A61B 5/113** $^{(2006.01)}$

(21) Application number: **06111715.6**

(22) Date of filing: **24.03.2006**

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**AL BA HR MK YU**

(71) Applicant: **Institut de Recherche en Réadaptation - Réinsertion**
**1950 Sion (CH)**

(72) Inventor: **Dériaz, Olivier**
**1950 Sion (CH)**

(74) Representative: **Ganguillet, Cyril et al**
**ABREMA**
**Agence Brevets & Marques**
**Ganguillet & Humphrey**
**Avenue du Théâtre 16**
**Case postale 5027**
**1002 Lausanne (CH)**

(54) **Indirect measurement of energy expenditure**

(57) The present invention relates to a method for determining and/or estimating the energy expenditure of an individual, a device suitable for indirectly measuring the energy expenditure of an individual and the use of textiles comprising piezoresistive materials in methods for determining an individual's energy expenditure.

# Figure 1

P1
P2
P3
P4
P5

EP 1 836 959 A1

## Description

The present invention relates to a method for determining and/or estimating the energy expenditure of an individual, a device suitable for indirectly measuring the energy expenditure of an individual and the use of textiles comprising piezoresistive materials and, optional, additional sensors, in methods for determining an individual's energy expenditure.

## Background of the Invention and Problem to be Solved

Human energy expenditure is an important variable to measure in several domains like research, nutrition, sport, rehabilitation, obesity etc. Several methods have been developed in order to measure this variable, which are shortly summarized below.

The most logical measurement of energy expenditure is the direct calorimetry method, which involves the direct assessment of the heat loss by measuring the evaporation, radiation convection and conduction of a human body. Unfortunately, this method is expensive and requires an uncomfortable, sophisticated and expensive device (i.e. a direct calorimeter). Moreover, the heat stored in the body (with an increase in body temperature) is not detected by this method. The only way to overcome this problem is to perform measurements during a long period of time with stable body temperature in order to be sure that the energy stored can be neglected.

Indirect calorimetry is based on the good relationship between the whole body oxygen consumption (measured at the mouth level) and the macronutrient oxidation. For all macronutrients oxidised (i.e. protein, carbohydrate and lipid) the energy produced when one litre of oxygen is consumed amounts from 19.5 kJ to 21.1 kJ. When none of these nutrients are stored, this method estimates also the relative proportion of macronutrient oxidised and, consequently, performs an accurate measurement of energy expenditure. However, this method is not comfortable for the subject because it requires a hermetic calorimetric chamber (in which the composition of the air of the ventilation is continuously measured) or a mask at the mouth level connected to gas analysers. In addition, this method is expensive and requires skills to be performed.

Energy expenditure can indirectly be assessed by the aid of double labelled water. A subject drinks a glass of water enriched with stable isotopes of hydrogen (deuterium) and oxygen ($^{18}$O). The hydrogen and oxygen are both eliminated with water (sweat and urines) whereas only oxygen is eliminated with the $CO_2$. Therefore, the difference in the decrease in $D_2$ and $^{18}$O (measured a week after) represents the $CO_2$ production rate of the body which is proportional to energy expenditure. Unfortunately double labelled water is expensive and isotope measurements require a mass spectrometer, which is expensive and difficult to use.

The linear relationship between energy expenditure and heart rate is the origin of another way of indirectly assessing energy expenditure. Heart rate is measured easily during a long period of time with cheap commercial equipment (Polar system). However, the relationship between heart rate and energy expenditure depends on the subjects and the training status as well as the type of exercise (arm or leg exercise). An accurate estimation of energy expenditure is difficult because it requires periodic assessment of its relationship with heart rate.

Accelerometers can also be used for estimating energy expenditure. Accordingly, a 3-D accelerometer can be placed close to the centre of mass of the body (i.e. the lumbar column). By a double integration, vertical body displacements can be measured in order to determine energy expenditure. This method, however, only registers displacements at the centre of mass. It thus entails the disadvantages that uniform horizontal displacements (speed), limb displacements without changes in centre of mass (internal work) as well as accelerations due to external environment (i.e. when the subject is in a car, on a bicycle etc.) are not measured. Consequently, several corrections have to be performed in order to estimate energy expenditure.

In view of the disadvantages encountered with the above methods for determining, directly or indirectly, energy expenditure, it becomes an objective of the present invention to obtain an accurate estimation of an individuals energy expenditure in a non-invasive, inexpensive, quick and easy way. It is thus an objective of the present invention to accurately assess energy expenditure without employment of an expensive equipment or equipment restraining the liberty of moving of an individual.

In particular, it is an objective of the present invention to provide means and/or methods for estimating and/or indirectly assessing energy expenditure, which can be employed by an individual in the absence of trained personal, and which do not impede the individual in every days activities. It is thus an objective of the present invention to find ways of assessing an individual's energy expenditure while working, exercising, eating, drinking and/or resting, for example.

## Summary of the Invention

Remarkably, the present inventors found that energy expenditure could be indirectly measured in a non-invasive manner that can be conducted over a prolonged time, ranging from minutes to several days or even longer, by measuring changes in thoracic and/or abdominal dimensions during respiration, preferably with the aid of textiles sensitive to changes in thoracic and/or abdominal dimensions.

Accordingly, in a first aspect, the present invention provides a method for indirectly measuring the energy expenditure of an individual, the method comprising the steps of:

- measuring, over a period of time, changes in thoracic and/or abdominal dimensions of the individual;
- determining, from the changes in thoracic and/or abdominal dimensions obtained in the preceding step, respiratory air movement of the individual; and,
- calculating, from the respiratory air movement, and, optionally, from other measured physical and/or physiologic parameters of the individual's body, the energy expenditure of the individual.

In another aspect, the present invention provides a device for indirectly measuring the energy expenditure of an individual, the device comprising:

- a device capable of measuring changes in thoracic and/or abdominal dimensions of the individual;
- at least one data processing unit, capable of determining, from the changes in thoracic and/or abdominal dimensions obtained in the preceding step, respiratory air movement of the individual and of determining, from the air movement, and, optionally other parameters, the energy expenditure of the individual.

In a further aspect, the present invention provides the use of textiles comprising piezoresistive materials in methods for determining an individual's energy expenditure through the, optionally indirect, measurement of respiratory air movement.

In the figures,

**Figure 1** illustrates the possible arrangement of stretch-sensitive strings of textiles in a shirt capable of measuring changes in thoracic and/or abdominal dimensions of the individual. The shirt comprises horizontally arranged strings named P1-P5 or, and a vertically extending strings.

**Figure 2** illustrates in further detail the horizontal and vertical stretch-sensitive strings of the shirt of Figure 1, P1-P5 and D1-D4, respectively.

## Detailed Description of the Preferred Embodiments

The present invention relates in one aspect to a method for indirectly measuring energy expenditure of an individual. Indirect measurement, for the purpose of the present invention, is a measurement in which the energy expenditure of an individual is not measured directly, as would be the case with direct calorimetry, as described above, for example. In contrast, indirect measurement refers to the measurement of physiological and/or physical parameters of and/or on the individual's body, which are then used to determine the energy expenditure by approximation, generally including calculation and optional assumptions as well as the actually measured parameters. It is the objective of the present invention to measure the physiological and/or physical parameters in a non-invasive way, which, in addition, does not or only to a limited extend confine the individual's habitual everyday activities.

The energy expenditure is an important variable in humans, and the method of the present invention will thus principally be used with human individuals. However, the present invention may equally be worked with animals, for example mammals. One could for example envisage biological studies with pets, such as dogs or cats, or other animals, in which energy expenditure of an animal is indirectly measured with the method of the present invention. Similar studies with livestock (cattle, etc) may be interesting in scientific or economical considerations.

The word "comprise" or "comprising", for the purpose of the present invention is intended to mean "including amongst other". It is not intended to mean "consisting only of".

The present invention comprises the step of measuring, over a period of time, preferably over a comparatively long period of from 30 minutes to several days, changes in thoracic and/or abdominal dimensions of the individual. The period may be short, that is, only 5 minutes, for example. Preferably, however, the period over which the measurement is performed is > 15 minutes, more preferably > 20 minutes, even more preferably > 30 minutes. For example, the period is 1 hour to 10 weeks, preferably 2 hours to 10 days and most preferably 3 to 48 hours. The period may be interrupted, which is especially useful if measurement is conducted over a period of several days. It is also possible, of course, to repeat measurements. It is an important advantage of the present invention that the measurement can be continued of periods longer than 15 or 30 minutes for example, because at longer periods of measurement the finally determined value of energy expenditure becomes closer to the actual energy expenditure of the individual. Since the present invention uses respiratory air movement as a key parameter for determining energy expenditure, fluctuations in respirations are balanced over longer periods and the measurement becomes more accurate.

According to a preferred embodiment of the present invention, changes in thoracic and/or abdominal dimensions of an individual are measured by the aid of extensile textiles that are applied on and/or around the individual's thorax and/or abdomen. During respiration, the extensile textile is stretched and the extent of stretching and/or contracting is measured.

Preferably, the extensile textile is arranged on the individual's thorax and/or abdomen in a way that the textile is measurably stretched and contracted substantially horizontally around at least part of the thorax and/or abdomen during respiration. Alternatively, the extensile textile is arranged to be subjected to vertical stretching and/or contraction on the individual's thorax and/or abdomen during respiration. It is, of course, possible to arrange the stretchable and/or contractive part of the textile in any way suitable to measure changes in thoracic and/or abdominal dimensions during respiration. For example, horizontal and vertical changes in dimensions may be measured through stretching and/or contraction of the extensile textile, and the textile may be arranged on the individual's body accordingly.

According to a preferred embodiment of the present invention, the changes in thoracic and/or abdominal dimensions are measured by the aid of textiles comprising piezoresistive materials. Accordingly, the device capable of measuring changes in thoracic and/or abdominal dimensions of the individual preferably comprises textile comprising piezoresistive materials.

Examples of textiles comprising piezoresistive materials are "Smart Textiles", which are commercially available, for example from the companies Sensatex Inc. (www.sensatex.com). SOFTswitch Ltd. (www.softswitch.co.uk), and Smartex (www.smartex.it). Smart textiles may be processed to wearable devices, for example typical clothes, such as tight-fitting shirts, for example. Wearable devices are capable of measuring many physiological parameters due to piezoresistive materials embroiled on or within the textile. Further technical information may be derived from the indicated web-sites, for example. Piezoresistive materials have the property of changing their electrical resistance upon mechanical stress, such as stretching, for example. From the change in electrical resistance, changes in thoracic and/or abdominal dimensions can be derived.

The processing unit in the device of the present invention preferably receives inputs from the piezoresistive materials and/or additional, optional sensors and translates the inputs in changes in thoracic and/or abdominal dimensions. These changes are constantly received by the processing unit over time and can thus also be used to measure respiratory frequency (f), which corresponds to the number of stretching and contractions per time unit.

The skilled person will understand that the term "measuring changes in thoracic and/or abdominal dimensions" does not necessarily require that a given extension of the thorax during inhalation, for example, needs to be measured in terms of mm and or cm. However, this expression means that a value or a signal is obtained, which is dependent on the extent of extension of the thorax and/or abdomen, during exhalation, for example. Accordingly, in respiration, a signal is produced, such as a change in electric resistance, in case of Smart textiles, for example, which can be interpreted as an indication of the extent of the change of thoracic and/or abdominal dimension. This signal may then be used in a further process step to determine respiratory air movement.

Preferably, the device of the invention further comprises a memory unit. The memory unit is preferably used to register the inputs of the piezo-resistive material, optionally translated to changes in thoracic and/or abdominal dimensions. In this way, the processing unit is capable to determine, from a sum of all respiratory changes in dimensions and from the respiratory frequency the overall respiratory air movement.

The device of the present invention preferably comprises, or may be connected, wired or wireless, to a data input unit. This data input unit can thus be located on a remote location. For example, the data input unit may be located on an internet server. The data input unit preferably receives physical and/or physiological data, for example, concerning the specific individual the energy expenditure of whom is indirectly measured. Accordingly, gender, age, body height, body weight, body mass index, time of the period of assessment, correction factors and so forth may be entered and forwarded to the data processing unit and, optionally the memory unit. These factors may be used to optimise algorithms for calculating energy expenditure from measured physical and/or physiological parameters, for example.

The device of the present invention may comprise at least one output unit, for example a display unit, such as a monitor, a screen, a printer, a loudspeaker. The input and output unit, if present, may be constituted by one single device, a touch-sensitive screen, for example. It is also possible that the output unit receives, for example in a wireless arrangement, the processed data from the remote data input unit. The output unit preferably indicates the individual energy expenditure. The output unit may also used to display intermediate results in the calculation of energy expenditure, for example, as well as the data entered through the input unit. Given that the device of the present invention is preferably user-friendly, the device, and in particular the input area, can preferably be used by non-skilled persons, for example the individual her-/ himself.

Preferably, the parts of devices carried by an individual during measurements have a weight of < 10 kg, preferably > 5 kg, more preferably < 3 kg, even more preferably < 2 kg and most preferably < 1 kg. The parts of the device carried by the individual during measurement include, for example extensile textiles, sensors for registering change of dimensions, optional sensors for measuring $pCO_2$ and/or $O_2$, and optional senders suitable to send data to a data processing unit, for example.

A more detailed description of how changes in thoracic and/or abdominal dimensions may be measured by Smart textiles is reported by G- Loriga, N. taccini, D. De Rossi and R. Paradiso in « Textile Sensing Interfaces for Cardiopulmonary Signs Monitoring », Proceedings of the 2005 IEEE, Engineering in Medicine and Biology 27th Annual Conference, Shanghai, China. In this publication, also the measurement of the respiratory frequency is disclosed.

The method of the present invention comprises the step of determining, from the changes in thoracic and/or abdominal dimensions obtained in the preceding step, respiratory and/or air movement of the individual.

Respiratory air movement, for the purpose of the present invention, refers to any movement of air for respiratory purposes. Accordingly, the term, as used herein, generally refers to "ventilation" or "pulmonary ventilation", and is determined by calculation from the changes in dimensions of the thorax and/or the abdomen and from the respiratory frequency. The unit of this physiological parameter is generally volume of air per time unit. However, the term includes the possibility of calculating energy expenditure of an individual in the absence of the determination on of a concrete value for pulmonary ventilation. For example, it can be envisaged that the changes of thoracic and/or abdominal dimensions are used to determine the alveolar ventilation only, and deduce the energy expenditure from this value. Alternatively, the changes in thoracic and/or abdominal dimensions may be translated directly to a value related in some way to ventilation for determining energy expenditure. The term respiratory air movement thus refers to any movement that lead to changes in the thoracic and/or abdominal dimensions and which is related to and/or at the origin of the rate of gas exchange in an individual's lungs.

Depending on the measurements of changes in thoracic and/or abdominal dimensions, any suitable algorithm may be established by the skilled person for translating the changes in dimensions to ventilation. The algorithm may be based on comparison of the changes in dimensions with results obtained by the aid of a portable spirometer. Accordingly, an algorithm may be established for any individual specifically. Alternatively, a generalised algorithm may be established by averaging a number of measurements comparing actual respiratory air movement and the signal of the piezo-resistive material, for example, taken from different individuals. In this way a generalised algorithm may be used, which approximates the respiratory air movement from changes in thoracic and/or abdominal dimensions.

An illustration of a simple algorithm suitable for determining respiratory air movement is reproduced below. In this algorithm it is assumed, for the sake of simplicity, that the abdomen and thorax have a cross-section of an ellipse having a small radian r, and a large radian r • k, with k being a constant, for example about 2.

In this algorithm, the perimeter, P, of the ellipse equals $\pi \cdot r \cdot (1+ k)$, and, the surface area, A, of the ellipse is $\pi \cdot r^2 k$, or, $A = (P^2 \cdot k)/ (\pi \cdot (1+ k)^2)$.

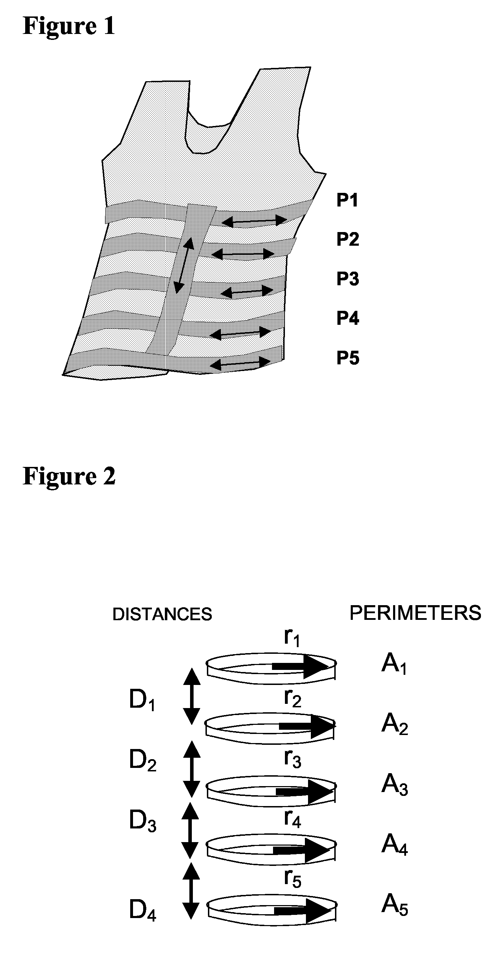

Based on the device illustrated in Figure 1, equation 1 below permits calculating the change in volume between the end of an inspiration and the end of an expiration, in which each segment of the shirt is assimilated as a sum of truncated cones in which the cross-sectional areas are calculated from the perimeters:

$$\Delta V_{\text{t-shirt}} = k_o \bullet \underbrace{\sum_{i=1}^{n-1} (P_i^2 - Q_i^2 + P_{i+1}^2 - Q_{i+1}^2 + P_i \bullet P_{i+1} - Q_i \bullet Q_{i+1}) \bullet D_i/3}_{\Delta \text{ stretch}} \qquad \text{equation 1}$$

in which:

- $\Delta V$ t-shirt is maximal difference in volume during a respiration cycle ($cm^3$)
- $\Delta$ stretch represents the variable part of the equation (the sum of n-1 truncated cones at the inspiration and expiration)
- P is the perimeter of a textile string in the shirt of Figure 1 at the end of an inspiration (cm)
- Q is the perimeter of the textile string at the end of an expiration (cm)
- D is the distance between 2 strings as illustrated in Figure 2 (length).
- n is the number of strings
- $k_o = k/[\Pi \bullet (1+k)^2$ represent the constant part of the equation in order to calculate the areas from the perimeters. It is assumed that k is constant through all sections of the trunk.

For adapting the change in volume calculated above to the actual change in volume a correction factor may be established, which is obtained by comparing the calculated volume to the change of volume actually observed by using a spirometer, for example. This is illustrated in equations 2 and 3 below,

$$VT = k_1 \bullet \Delta V_{\text{t-shirt}} \qquad\qquad \text{equation 2}$$

$$VT = k_1 \bullet k_o \bullet \Delta\text{stretch} = C \bullet \Delta\text{stretch} \qquad\qquad \text{equation 3}$$

in which:

- VT is the volume measured with a portable spirometer (volume)
- $\Delta$stretch is a part of equation 1 (volume)
- $k_1$ is a correction factor ($k_1 \approx 1$)
- $C = k_1 \cdot k_o$ is the correction factor determined by performing a correlation between t-shirt measurements and the spirometric measurements. Practically, $k_1$ and $k_o$ do not need to be calculated individually.

For determining respiratory air movement according to this algorithm, the total volume measured over a period of time has to be divided by the time of one respiration cycle (t) or multiplied by the respiratory frequency (f), as illustrated in equation 4,

$$\dot{V}T = C \bullet \Delta\text{ stretch} \bullet f \qquad\qquad \text{equation 4}$$

in which,

- $\dot{V}T$ represents the ventilation (volume/ time)
- f the respiration frequency (respiration cycles / time)
- $\Delta$stretch is the result obtained from the t-shirt measurement (volume)

Equations 1-4 thus exemplify an algorithm for determining, by calculation, respiratory air movement from the measurement of changes in thoracic and/or abdominal dimensions over time. While this algorithm, for the sake of comprehension, expressly includes the measurement of changes in dimensions in terms of metric units, this is not mandatory, as indicated above already: a signal or a number of signals obtained from Smart Textiles during respiration may, by a suitable algorithm, directly be translated to respiratory air movement. This signal, however, generally needs to be in correlation to the changes in thoracic and/or abdominal dimensions during respiration.

Preferably, the values for respiratory air movement obtained from measuring changes of thoracic and/or abdominal dimensions are calibrated. Preferably, they are calibrated by adjusting to values obtained in parallel (at the same time period) to the measurement of the changes of dimensions. The values obtained in parallel are preferably obtained by measuring, over a relatively short period, for example 0.5 to 10 min, the volume of the expired and/or inspired air of the individual, for example by a spirometer. Preferably, calibration is realised by comparing the values obtained from the changes in dimensions with values obtained from measuring volume of expired air over the same time period, and, deducing, from the comparison an algorithm suitable to transform the values obtained from measuring changes in thoracic and/or abdominal dimensions to respiratory air movement, in to particular pulmonary and/or alveolar ventilation.

The method of the present invention further comprises a step of calculating, from the respiratory air movement, and, optionally, from other measured physiologic parameters, the energy expenditure of the individual.

In a preferred embodiment of the present invention, the energy expenditure of the individual is indirectly assessed from its respiratory air movement (ventilation). Accordingly, Amasa B. Ford and Herman K. Hellerstein, J. Appl. Physiol. 14(6); 891-893, 1959 showed the correlation between pulmonary ventilation and energy expenditure. According to this embodiment measurements of further physiological parameters are not required. As indicated before, the accuracy of the estimation of energy expenditure through ventilation increases with the length of the period over which ventilation is measured. Over longer periods of time, errors in the calculation due to changes in ventilation not associated with metabolism can be minimized. Thanks to the fact that the present invention is operable in the absence of heavy or otherwise obstructive devices, the prolonged measurement can conveniently be effected through the method of the present invention, and an accurate indication of an individual's energy expenditure can be obtained.

According to another embodiment, the method according to the present invention comprises further, non-invasive steps for indirectly measuring energy expenditure, wherein the finally determined and/or estimated value of overall energy expenditure is calculated from the energy expenditures obtained by several non-invasive steps of indirectly measuring energy expenditure.

For example, assessment of $CO_2$ output through respiration is closely related to the energy metabolism and may thus me determined and combined, by a suitable algorithm, with the energy expenditure derived from respiratory air movement alone. Accordingly, Itoh M. et al., Japanese Journal of Physiology, 52, 489-496, 2002 have demonstrated the close relationship between $CO_2$ output and energy metabolism. Interestingly, this relationship does not depend on the training status and the type of exercise (i.e. arm or leg exercise).

Alternatively, or in addition to that, energy expenditure may be determined from measuring heart frequency, which is also closely related to energy expenditure.

Accordingly, respiratory air movement, and, in addition, $CO_2$ production rate and/or heart frequency may be adduced to determine energy expenditure through different measurements and algorithms, for example. According to the present invention, an approximate, balanced value for an individual's energy expenditure may be derived by taking into account one, a combination of two, or all three of the aforementioned parameters.

According to a preferred embodiment of the present invention, an optional other measured physiological parameter is the capillary and/or arterial $CO_2$ partial pressure. The capillary $CO_2$ partial pressure (may be used in order to estimate $CO_2$ production rate and, as indicated above, provides a possibility of indirectly measuring energy expenditure. Preferably, capillary $O_2$ partial pressure is also measured.

Accordingly, the device of the present invention preferably comprises a unit for measuring capillary and/or arterial $CO_2$ partial pressure. Results of this measurement, optionally submitted to calculation, are preferably sent to the data processing unit and, if adequate, stored in the optional memory unit. Preferably, capillary, arterial and/or alveolar $CO_2$ contents or partial pressures may be indicated in the optional display unit, for example upon request from a person handling the device of the present invention.

According to a preferred embodiment, the method of the present invention comprises the steps of determining, by non-invasive measurement, the individual's capillary and/or arterial $CO_2$ partial pressure ($pCO_2$); determining the individual's $CO_2$ production rate from parameters involving the capillary and/or arterial $pCO_2$, the respiratory air movement and, optionally, a pulmonary dead space of the individual; and, determining the individual's energy expenditure from parameters including the $CO_2$ production rate. For the purpose of the present invention, the arterial and the alveolar $CO_2$ partial pressure are supposed to be substantially identical and the two terms thus exchangeable, for the purpose of estimation. In case that with a given individual the respective values turn out not to be substantially different, the skilled person will readily be able to establish a suitable correction factor or to use another algorithm than the one proposed herein for indirectly assessing energy expenditure.

Devices for measuring capillary and/or arterial $CO_2$ and/or $O_2$ partial pressure are commercially available. Today, portable devices exist, for example from Linde Medical Sensors, Basel, Switzerland.

According to the present invention, simplified and lighter versions of such devices may be used, devoid of the equipment for "arterialisation" of the capillaries. In the heavier versions of these devices, capillaries are "arterialised" by heating, which permits direct measuring of arterial partial pressures of $CO_2$ and $O_2$, for example. Unfortunately, the equipment required for heating is often heavy and encumbering. As indicated further below, arterial $CO_2$ and may be determined by algorithm from capillary $CO_2$, an optionally $O_2$, partial pressure, by making certain general assumptions or using constants applicable for healthy individuals in general, for example.

Any algorithm for approximating energy expenditure based on measurements of capillary and/or arterial $CO_2$ and $O_2$ partial pressure may be established by the skilled person. Such an algorithm may or may not involve values derived from measurement of respiratory air movement. These algorithms, as those used for calculating energy expenditure from respiratory air movement, may be established based on theory and/or available approximations and, optionally, improved based on consideration of parameters specific to the individual (age, sex, body size, etc.) as indicated above with respect to the device of the present invention.

A possible algorithm for indirectly measuring energy expenditure based on measurement of capillary $CO_2$ and $O_2$ partial pressure may be based on equation 5 below, in which energy expenditure ($\dot{E}$) is correlated to $CO_2$ output ($\dot{V}CO_2$) and an estimated energy equivalent of $CO_2$ ($EECO_2$):

$$\dot{E} = \dot{V}CO_2 \bullet EECO_2 \qquad\qquad \text{equation 5}$$

Depending on the macronutrients oxidized (proteins, fats or carbohydrates), varying amounts of $O_2$ and $CO_2$ are consumed and produced, respectively. For long periods of time (i.e. days), it can be reasonably assumed that the proportion of macronutrient oxidized is relatively stable. Therefore, and for the sake of convenience, an $EECO_2$ of about 24 kJ/ L $CO_2$ can be used for calculations.

A more accurate value of $EECO_2$ may be determined for any specific individual, for example by assessing factors like nutrient intake, body composition (for instance body mass index), intensity of exercise etc.

The other parameter necessary to calculate energy expenditure based on equation 5 is the $CO_2$ production rate ($\dot{V}CO_2$). Below an algorithm for approximating $\dot{V}CO_2$ from measurements or estimation of capillary $CO_2$ and $O_2$ partial pressures in the absence or arterialisation is provided for illustration.

According to a preferred embodiment of the method of the present invention, the individual's energy expenditure is determined by calculation from parameters including the partial pressure of alveolar and/or arterial $CO_2$, the barometric pressure and the individual's respiratory air movement.

$\dot{V}CO_2$ can be calculated by equation 6 (JB, West, Respiratory physiology, ©1974, 1975, The Williams and Wilkins Company, Baltimore).

$$\dot{V}CO_2 = \dot{V}A \bullet PaCO_2/Pb \qquad\qquad \text{equation 6}$$

Where:

- $\dot{V}A$ is the alveolar ventilation (mL/min)
- $\dot{V}CO_2$ is the $CO_2$ production rate (mL/min)
- Pb is the barometric pressure (mmHg)
- $PaCO_2$ = is the alveolar (generally about equal to arterial) $CO_2$ pressure (mmHg)

$\dot{V}A$ may be determined by using the respiratory air movement (ventilation) assessed above by equations 4, for example. Accordingly, it may be determined from equation 7:

$$\dot{V}A = \dot{V}T - \dot{V}D \qquad\qquad \text{equation 7}$$

Where :

- $\dot{V}A$ is the alveolar ventilation (mL/min)
- VD is the physiological dead space ventilation (mL/min)
- VT is the ventilation (mL/min), which can be taken from equation 4 above.

More details on equations 4 to 7 may be derived from the textbook of J.B. West, Respiratory physiology, ©1974,1975, The Williams and Wilkins Company, Baltimore.

The anatomic dead space may be used as an approximation of the physiological dead space and is derived from the following formula (Hart, MC et al, J Appl Physiol 18: 519-522, 1963):

$$VD = 7.585 \bullet Ht^{2.363} \bullet 10^{-4} \qquad\qquad \text{equation 8}$$

Where:

- VD is expressed in ml
- Ht is the body height expressed in cm

Of course, anatomic dead space may also be determined more accurately with respect to a specific individual.

Alveolar ventilation may, in summary, be determined from the ventilation derived from the measurements of changes in dimensions of the thorax and/or abdomen, and other parameters, which are easily accessible or which may be determined more accurately, at the discretion of the skilled person, or the person working the method of the present invention. Alveolar ventilation may then be used, along with arterial $CO_2$ pressure, for assessing $CO_2$ production rate.

Accordingly, the other variable in the algorithm for assessing $CO_2$ production rate is arterial $CO_2$ pressure, a variable that is relatively constant in healthy individuals (about 40 mmHg). Calculations with alveolar ventilation and arterial $CO_2$ pressure approximated by measurement may, of course, be adduced to improve the prediction equation without any supplementary material.

Various ways may be employed for estimating or measuring this variable, such as by arterialisation of capillaries and subsequent measurement of capillary $CO_2$ partial pressure, as described above. Preferably, the arterial $CO_2$ pressure is determined non-invasively and in the absence of heavy equipment that could be perceived as a hindrance by the individual the energy expenditure of whom is assessed. Accordingly, arterial $CO_2$ partial pressure, which is generally assumed to be equal to alveolar partial $CO_2$ pressure, may be derived from an individual's capillary $CO_2$ and/or $O_2$ partial pressures.

Accordingly, general assumptions may be made and general algorithms may be used to derive arterial partial pressure from capillary $CO_2$ and/or $O_2$ partial pressures. As before, any assumption may be made more accurate by approximating constants and/or simplifications to values more accurate for a specific individual's. Accordingly, partial pressure of a given gas in blood, for example, may be determined by algorithm in which several variables are included like dissociations curves, skin temperature etc. This topic has been largely described by the group of J.W Severinghaus in san Fransisco California.

**Preferred Modes of Carrying out the Invention**

The invention will now be described for mere illustrative but not limitative purposes in the contemplative examples below, in which abbreviations have the usual meaning in the art.

Example 1: Determining Respiratory Air Movement with Smart Textile

With a healthy (male or female) individual, changes in thoracic and abdominal volume are measured by the aid of a commercially available Smart Textile obtained from Smartex (www.smartex.it). Measurements are taken independently from five horizontal perimeters P1 - P5, each at a distance of 10 cm from the preceding, as illustrated in Figure 1. In addition, vertical changes in dimensions are measured by a single, vertically extending textile string, which is divided in 4 parts, D1-D4, each lying in between two perimeters (Figure 1).

Changes in volume are determined assuming that the cross-section of the thorax and abdomen has the shape of an ellipse. Equation 1 in the description is used to calculate thoracic/ abdominal volume of an individual over time based on the measurements in Table 1 below:

**Table 1:** Measurements of Changes in Thoracic and Abdominal Dimensions

| i | $P_i$ | $P_i^2$ | $Q_i$ | $Q_i^2$ | $D_i$ | $k/[\pi\bullet(1+k)^2]$ |
|---|---|---|---|---|---|---|
| 1 | 90.0 | 8100.0 | 88.5 | 7832.3 | 10.0 | 0.0597 |
| 2 | 90.0 | 8100.0 | 88.5 | 7832.3 | 10.0 | 0.0597 |
| 3 | 90.0 | 8100.0 | 88.5 | 7832.3 | 10.0 | 0.0597 |
| 4 | 90.0 | 8100.0 | 88.5 | 7832.3 | 10.0 | 0.0597 |
| 5 | 90.0 | 8100.0 | 88.5 | 7832.3 | | 0.0597 |

In Table 1, i is the measurement number, P is the perimeter of the textile string at the end of an inspiration (cm), Q is the perimeter of the textile string at the end of an expiration (cm), D is the distance between 2 strings (cm), k is a constant (in this example, k takes the value of 2),

Table 2 illustrates the calculation of the change of volume based on the measurements obtained from the Smart Textile above.

**Table 2:** Calculation of Change of Volume from Changes in Thoracic and Abdominal Dimenisons

| i | $P_i^2-Q_i^2$ | $P_{i+1}^2-Q_{i+1}^2$ | $P_i\bullet P_{i+1}$ | $Q_i\bullet Q_{i+1}$ | $P_i\bullet P_{i+1}-Q_i\bullet Q_{i+1}$ | $S=P_i^2-Q_i^2+P_{i+1}^2-Q_{i+1}^2+P_i\bullet P_{i+1}-Q_i\bullet Q_{i+1}$ | $\Delta V_{t\text{-shirt}}=S\bullet k_o\bullet D_i/3$ |
|---|---|---|---|---|---|---|---|
| 1 | 267.8 | 267.8 | 8100.0 | 7832.3 | 267.8 | 803.3 | 159.80 |
| 2 | 267.8 | 267.8 | 8100.0 | 7832.3 | 267.8 | 803.3 | 159.80 |
| 3 | 267.8 | 267.8 | 8100.0 | 7832.3 | 267.8 | 803.3 | 159.80 |
| 4 | 267.8 | 267.8 | 8100.0 | 7832.3 | 267.8 | 803.3 | 159.80 |
| Σ= | **1071** | **1071** | **32400** | **31329** | **1071** | **3213** | **639.21** |

In Table 2, $\Delta V_{t\text{-shirt}}$ is the maximal difference in volume during a respiratory cycle, $k_o$ is equal to $k/[\pi\bullet(1+k)^2]$,

and the other symbols have the same meaning as in Table 1.

Assuming that the correction factor $k_1$ (equations 2 and 3 in the description) is 1, the present contemplative example determines a ventilation (= respiratory air movement) of $VT = \Delta V_{t-shirt} = 640$ ml/min.

This result may be compared to results obtained from direct measurement with a spirometer. A correction factor will generally be calculated as described in equations 2 and 3 of the description.

Example 2: Indirect Determination of Energy Expenditure Based on Respiratory Air Movement

Following the observations of Amsa et al (1959), it can be calculated that a ventilation of $640 \cdot 12 = 7.68$ L·min-1 corresponds to energy expenditure (E) of $-0.26 + 0.204 \cdot 7.68 = 1.31$ kcal per minute or 1880 kcal per day.

The accuracy of this prediction of the energy expenditure determination can be sufficient in order to predict, in a given individual, a change in energy expenditure through time. Accordingly, energy estimation of an individual is indirectly measured in a non-invasive manner, not hindering the individual in every-days activities.

Example 3: Calculation of CO2 production rate

The CO2 production rate can be calculated according to the following equations, based on equation 6 of the description, modified by the presence of a correction factor $C_{STPD}$ to express $\dot{V}CO_2$ in STPD conditions (STPD = standard temperature (273 K) and pressure of dry gas).

$$\dot{V}CO_2 = \dot{V}A \bullet PaCO_2/Pb \bullet C_{STPD} = (\dot{V}T.\dot{V}D) \bullet PaCO_2/Pb \bullet C_{STPD}.$$

The dead-space can be determined by measurement or approximated by calculation. In the present example, the dead space is determined by calculation for a human subject of 179 cm body height, and is $7.585 \bullet 179^{2.363} \bullet 10^{-4} = 160$ ml.

Atmospheric pressure is measured to be 760 mmHg. The correction factor $C_{STPD}$ is then calculated as follows (see J.B. West, Respiratory physiology, ©1974, 1975, The Williams and Wilkins Company, Baltimore):

$$C_{STPD} = 273/310 \bullet (720 - 47)/760 = 0.78$$

If the respiratory frequency amounts to 12 per minute, the $\dot{V}CO_2$ for the individual can be calculated as follows:

$$\dot{V}CO_2 = 480 \bullet 40/720 \bullet 12 \bullet 0.78 = 250 \text{ ml } CO_2 \text{ per minute.}$$

$PaCO_2$ was considered to be equal to 40 mmHg.

Example 4: Estimation of Energy Expenditure Based on $CO_2$ Production Rate

Depending on the macronutrients oxidized (proteins, lipids or carbohydrates), the $CO_2$ production varies. Over long periods of time, the proportion of macronutrients oxidized is stable, and an energy equivalent of produced $CO_2$ (EECO2) of 24 kj/L $CO_2$ is taken for this example.

Therefore, energy expenditure of the individual ($\dot{E}$) can be calculated from the $CO_2$ production rate obtained in Example 5 as follows:

$$\dot{E} = \dot{V}CO_2 \bullet EECO_2 = 0.250 \bullet 24 = 6 \text{ kJ/min (2065 kcal per day).}$$

Accordingly, the individual of the contemplative Example 1 has an energy expenditure of 2065 kcal per day.

**Claims**

1. A method for indirectly measuring the energy expenditure of an individual, the method comprising the steps of:

   - measuring, over a period of time, changes in thoracic and/or abdominal dimensions of the individual;
   - determining, from the changes in thoracic and/or abdominal dimensions obtained in the preceding step, respiratory air movement of the individual; and,
   - calculating, from the air movement, and, optionally, from other measured physical and/or physiologic parameters of the individual's body, the energy expenditure of the individual.

2. The method of claim 1, in which the changes in thoracic and/or abdominal dimensions are measured by the aid of extensile textiles that are applied on and/or around the individual's thorax and/or abdomen.

3. The method of claim 1, in which the changes in thoracic and/or abdominal dimensions are measured by the aid of textiles comprising piezoresistive materials.

4. The method of claim 1, comprising further, non-invasive steps for indirectly measuring energy expenditure, wherein the finally determined and/or estimated value of overall energy expenditure is calculated from the energy expenditures obtained by several non-invasive steps of indirectly measuring energy expenditure.

5. The method of any of the preceding claims in which one optional other measured physiologic parameter is capillary and/or arterial $CO_2$ and/or $O_2$ partial pressure.

6. The method of any of the preceding claims for indirectly measuring the individual's energy expenditure, comprising the steps of:

   - determining, by non-invasive measurement, the individual's capillary and/or arterial $CO_2$ partial pressure ($pCO_2$);
   - determining the individual's $CO_2$ production rate from parameters involving the capillary and/or arterial $pCO_2$, the respiratory air movement and, optionally, a pulmonary dead space of the individual; and,
   - determining the individual's energy expenditure from parameters including the $CO_2$ production rate.

7. The method of Claim 6, in which the individual's energy expenditure is determined by calculation from parameters including the partial pressure of alveolar and/or arterial CO2, the barometric pressure and the individual's respiratory air movement.

8. The method of any of the preceding claims, in which the individual's energy expenditure is further measured indirectly by the steps of:

   - measuring the individual's heart frequency; and
   - determining and/or estimating the individual's energy expenditure from the individual's heart frequency.

9. A device for indirectly measuring the energy expenditure of an individual, the device comprising:

   - a device capable of measuring changes in thoracic and/or abdominal dimensions of the individual;
   - at least one data processing unit, capable of determining, from the changes in thoracic and/or abdominal dimensions obtained in the preceding step, respiratory air movement of the individual and of determining, from the air movement, and, optionally other parameters, the energy expenditure of the individual.

10. The use of textiles comprising piezoresistive materials in methods for determining an individual's energy expenditure through the, optionally indirect, measurement of respiratory air movement.

# Figure 1

P1
P2
P3
P4
P5

# Figure 2

DISTANCES

PERIMETERS

$r_1$    $A_1$

$D_1$

$r_2$    $A_2$

$D_2$

$r_3$    $A_3$

$D_3$

$r_4$    $A_4$

$r_5$    $A_5$

$D_4$

**EP 1 836 959 A1**

**European Patent Office**

# EUROPEAN SEARCH REPORT

Application Number

EP 06 11 1715

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 86/04497 A (FLEXIGAGE LIMITED) 14 August 1986 (1986-08-14) * page 1, line 3 - line 18 * * page 2, line 34 - page 3, line 7 * * page 4, line 23 - page 7, line 29 * ----- | 1-4,8-10 | INV. A61B5/113 |
| A | US 4 960 118 A (PENNOCK ET AL) 2 October 1990 (1990-10-02) * the whole document * ----- | 2,3 | |
| D,A | LORIGA G ET AL: "Textile Sensing Interfaces for Cardiopulmonary Signs Monitoring" ENGINEERING IN MEDICINE AND BIOLOGY SOCIETY, 2005. IEEE-EMBS 2005. 27TH ANNUAL INTERNATIONAL CONFERENCE OF THE SHANGHAI, CHINA 01-04 SEPT. 2005, PISCATAWAY, NJ, USA,IEEE, 1 September 2005 (2005-09-01), pages 7349-7352, XP010908176 ISBN: 0-7803-8741-4 * the whole document * ----- | 1-3,9,10 | |
| | | | TECHNICAL FIELDS SEARCHED (IPC) |
| D,A | FORD, AMASA B.; HELLERSTEIN, HERMAN K.: "Estimation of energy expenditure from pulmonary ventilation" J APPL PHYSIOL, vol. 14, no. 6, 1959, pages 891-893, XP009071952 * the whole document * ----- | 1,9,10 | A61B |

The present search report has been drawn up for all claims

| Place of search | Date of completion of the search | Examiner |
|---|---|---|
| Munich | 12 September 2006 | Hooper, Martin |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

13

**EP 1 836 959 A1**

## ANNEX TO THE EUROPEAN SEARCH REPORT
## ON EUROPEAN PATENT APPLICATION NO.

EP 06 11 1715

This annex lists the patent family members relating to the patent documents cited in the above-mentioned European search report.
The members are as contained in the European Patent Office EDP file on
The European Patent Office is in no way liable for these particulars which are merely given for the purpose of information.

12-09-2006

| Patent document cited in search report | | Publication date | Patent family member(s) | | Publication date |
|---|---|---|---|---|---|
| WO 8604497 | A | 14-08-1986 | AU | 5393386 A | 26-08-1986 |
| | | | DK | 459986 A | 26-09-1986 |
| | | | EP | 0211880 A1 | 04-03-1987 |
| | | | ES | 8706420 A1 | 16-09-1987 |
| | | | JP | 62501541 T | 25-06-1987 |
| | | | US | 4966155 A | 30-10-1990 |
| US 4960118 | A | 02-10-1990 | NONE | | |

EPO FORM P0459

For more details about this annex : see Official Journal of the European Patent Office, No. 12/82

14

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- Textile Sensing Interfaces for Cardiopulmonary Signs Monitoring. **G- LORIGA ; N. TACCINI ; D. DE ROSSI ; R. PARADISO.** Engineering in Medicine and Biology 27th Annual Conference. IEEE, 2005
- **AMASA B. FORD ; HERMAN K. HELLERSTEIN.** *J. Appl. Physiol.,* 1959, vol. 14 (6), 891-893

- **ITOH M. et al.** *Japanese Journal of Physiology,* 2002, vol. 52, 489-496
- **JB, WEST.** Respiratory physiology. The Williams and Wilkins Company, 1974
- **J.B. WEST.** Respiratory physiology. The Williams and Wilkins Company, 1974
- **HART, MC et al.** *J Appl Physiol,* 1963, vol. 18, 519-522